# FASCICULE DE BREVET EUROPEEN

(11) **EP 2 800 601 B1**
(45) Date de publication et mention de la délivrance du brevet: **16.12.2015**
(21) Numéro de dépôt: 12775808.4
(22) Date de dépôt: 02.10.2012
(51) Int. Cl.: A61M 16/08, A61M 16/12, A61M 16/00

(54) **DISPOSITIF DE DISTRIBUTION DE GAZ À CIRCUIT EN BOUCLE ET RÉSERVOIR-TAMPON**
GASVERTEILUNGSVORRICHTUNG MIT EINEM STEUERKREIS UND PUFFERBEHÄLTER
GAS DISTRIBUTION DEVICE WITH A LOOP CIRCUIT AND BUFFER TANK

(30) Priorité: 03.01.2012 FR 1250033
(43) Date de publication de la demande: 12.11.2014
(73) Titulaire: L'Air Liquide Société Anonyme pour l'Etude et l'Exploitation des Procédés Georges Claude, 75007 Paris (FR)
(72) Inventeur: MARTIN, Andrew, Wilmington, DE 19805 (US); KATZ, Ira, 92190 Meudon (FR); DAVIET, Christian, 75011 Paris (FR); TEXEREAU, Joëlle, 75013 Paris (FR)
(74) Mandataire: Pittis, Olivier
(86) Numéro de dépôt international: PCT/FR2012/052227
(87) Numéro de publication internationale: WO 2013/102707

(56) Documents cités:
- EP-A1- 1 402 915
- WO-A1-01/74433
- WO-A1-2006/089427
- WO-A1-2007/012170

## Description

L'invention porte sur un dispositif de distribution de gaz médical apte à et conçu pour venir s'insérer entre une source de gaz thérapeutique ou médical et une interface patient, tel un masque respiratoire.

Pendant l'administration d'un gaz médical ou thérapeutique à un patient, l'entraînement intempestif d'air ambiant dans le flux de gaz envoyé au patient peut engendrer une dilution de la teneur en gaz médical et conduire à fournir au patient une teneur en gaz médical insuffisante car en deçà de sa concentration thérapeutique optimale, d'où il peut résulter une réduction partielle ou totale de l'effet bénéfique attendu.

Pour limiter ce phénomène d'entrées d'air parasites, lors de l'utilisation de systèmes d'administration sans récupération et recyclage des gaz expirés, il convient d'avoir une alimentation en gaz frais, c'est-à-dire un débit de gaz frais, supérieure ou égale à la demande en gaz du patient, encore appelée le volume minute patient.

Le problème est que, lorsque la demande du patient en gaz médical inhalé est importante, il est nécessaire d'assurer une fourniture en gaz thérapeutique à débit élevé, ce qui engendre une consommation importante de gaz médical et un coût élevé du traitement.

Pour y remédier, les documents EP-A-1402915, et Slessarev et al. ; Crit. Care Med.; 2006 Vol. 34, No. 3 ; propose un dispositif d'administration permettant de réaliser une alimentation séquentielle en gaz. Celui-ci comprend un circuit de gaz en boucle avec valve inspiratoire, valve expiratoire et valve de dilution, orifice d'alimentation en gaz frais, orifice de distribution de gaz au patient, orifice d'évacuation de gaz vers l'atmosphère et ballon-réservoir pour recevoir du gaz frais. Il vient s'insérer entre la ligne d'alimentation en gaz et l'interface patient, en particulier un masque nasal ou facial.

Toutefois, ce dispositif n'est pas totalement idéal car la valve de dilution présente en tant que dispositif de sécurité, permet à de l'air ambiant de pénétrer dans le circuit quand la demande en gaz du patient excède le volume de gaz frais disponible.

De là, pour éviter que la concentration distribuée au patient soit insuffisante, c'est-à-dire qu'elle ne soit pas celle souhaitée, du fait d'une dilution avec de l'air ambiant entré via la vanne de dilution, l'alimentation en gaz frais droit être suffisamment élevé pour toujours satisfaire à la demande patient et ce, malgré d'éventuelles fluctuations de cette demande. Ceci oblige à alimenter le dispositif avec un débit important de gaz et conduit inévitablement à des pertes de gaz.

Ces pertes sont en outre accentuées par le fait que ce dispositif ne permet pas de valoriser les gaz expirés pauvres en CO₂ mais encore riches en gaz médical, qui sont expirés en tout début de phase expiratoire par le patient.

D'autres documents, par exemple US-A-7360538, WO-A-2009127049 et WO 2010061173, enseignent des dispositifs respiratoires similaires.

Toutefois, ceux-ci ne sont pas non plus totalement idéaux car présentant également des inconvénients du même type, en particulier une perte inévitable des gaz expirés pauvres en CO₂ mais encore riches en gaz thérapeutique, qui sont expirés en tout début de phase expiratoire par le patient.

On connait par ailleurs les documents suivants :
- WO-A-2007/012170 décrivant un appareil respiratoire permettant d'induire des teneurs en gaz cibles en fin de phase expiratoires, en particulier des changements séquentiels rapides de teneurs en CO₂ et O₂. L'appareil comprend une valve expiratoire à sens unique et un réservoir-tampon de gaz.
- WO-A-2006/089427 enseignant un appareil permettant d'induire une hypoxie chez un individu, qui comprend un port respiratoire, un réservoir de gaz inspiratoire, des moyens d'apport d'oxygène et réservoir expériatoire avec un port de sortie, ainsi que des moyens de fourniture séquentielle de gaz et des moyens d'élimination du CO₂.
- WO-A-01/74433 portant sur un procédé de contrôle de circuit respiratoire avec récupération d'une partie des gaz expirés et leur renvoi vers l'entrée du circuit pour engendrer leur ré-inhalation. Le circuit est muni de valves de contrôle du passage de gaz.

Le problème qui se pose dès lors est de proposer un dispositif respiratoire amélioré permettant de réaliser une distribution séquentielle de gaz mais sans engendrer de consommation excessive de gaz, c'est-à-dire en conservant un débit d'alimentation en gaz frais aussi faible que possible et ce, malgré d'éventuels fluctuations, en particulier des pics, de consommation et permettant en outre de diminuer les pertes de gaz expirés pauvres en CO₂ mais encore riches en gaz médical, qui sont expirés en tout début de phase expiratoire par le patient.

La solution est alors un dispositif de distribution de gaz comprenant :
- un circuit de gaz en boucle comprenant une première portion de circuit reliée fluidiquement à une deuxième portion de circuit, ladite deuxième portion de circuit étant reliée à une troisième portion de circuit, et ladite troisième portion de circuit étant reliée fluidiquement à la première portion de circuit,
- la première portion de circuit comprenant une valve inspiratoire, la deuxième portion de circuit comprenant une valve expiratoire, et la troisième portion de circuit comprenant une valve de dilution,
- un orifice d'alimentation en gaz frais agencé sur le circuit de gaz entre la valve inspiratoire et la valve de dilution,
- un orifice de distribution de gaz au patient agencé sur le circuit de gaz entre la valve inspiratoire et la valve expiratoire, et
- un orifice d'évacuation de gaz agencé sur le circuit de gaz entre la valve expiratoire et la valve de dilution,
caractérisé en ce qu'un réservoir-tampon de gaz expiratoire est relié fluidiquement audit circuit de gaz entre les valves inspiratoire et expiratoire.

Selon le cas, le dispositif de distribution de gaz l'invention peut comprendre l'une ou plusieurs des caractéristiques techniques suivantes :
- il comprend en outre un réservoir-tampon de gaz frais relié fluidiquement audit circuit de gaz entre les valves inspiratoire et de dilution.
- la valve inspiratoire, la valve expiratoire et la valve de dilution sont des valves à sens unique.
- l'orifice de distribution de gaz est en communication fluidique avec une interface patient, en particulier un masque respiratoire nasal ou facial.
- le réservoir-tampon de gaz expiratoire est déformable.
- le réservoir-tampon de gaz expiratoire a un volume interne maximal compris entre 10 et 500 ml, de préférence entre 50 et 400 ml.
- le réservoir-tampon de gaz expiratoire a un volume interne ajustable compris entre 10 et 500 ml, de préférence entre 50 et 400 ml.
- le réservoir-tampon de gaz expiratoire a un volume interne ajustable par incréments compris entre 10 et 100 ml, typiquement entre 20 et 60 ml.

L'invention porte en outre sur une installation d'administration de gaz thérapeutique à un patient comprenant une source de gaz thérapeutique alimentant, via l'orifice d'alimentation en gaz frais, le circuit de gaz en boucle d'un dispositif de distribution de gaz selon l'invention, et une interface patient connectée fluidiquement à l'orifice de distribution de gaz agencé sur ledit circuit de gaz en boucle.

De préférence, la source de gaz thérapeutique est un ventilateur et/ou l'interface est un masque respiratoire.

La présente invention va être mieux comprise grâce aux explications suivantes données à titre illustratif et faites en références aux Figures annexées parmi lesquelles :
- la Figure 1a représente un schéma d'un premier mode de réalisation d'un dispositif respiratoire selon l'invention,
- la Figure 1b représente un schéma d'un second mode de réalisation d'un dispositif respiratoire selon l'invention,
- la Figure 2 représente un cycle respiratoire sans demande en gaz excessive du patient,
- la Figure 3 donne des mesures de concentration moyenne d'hélium administré à un patient en utilisant un dispositif du commerce et, à titre comparatif, un dispositif selon la présente invention, et
- la Figure 4 schématise les variations de la concentration d'hélium mesurée en temps réel, pendant l'inspiration, avec administration de He/O₂ au moyen d'un dispositif selon la présente invention et, à titre comparatif, d'un dispositif selon l'art antérieur.

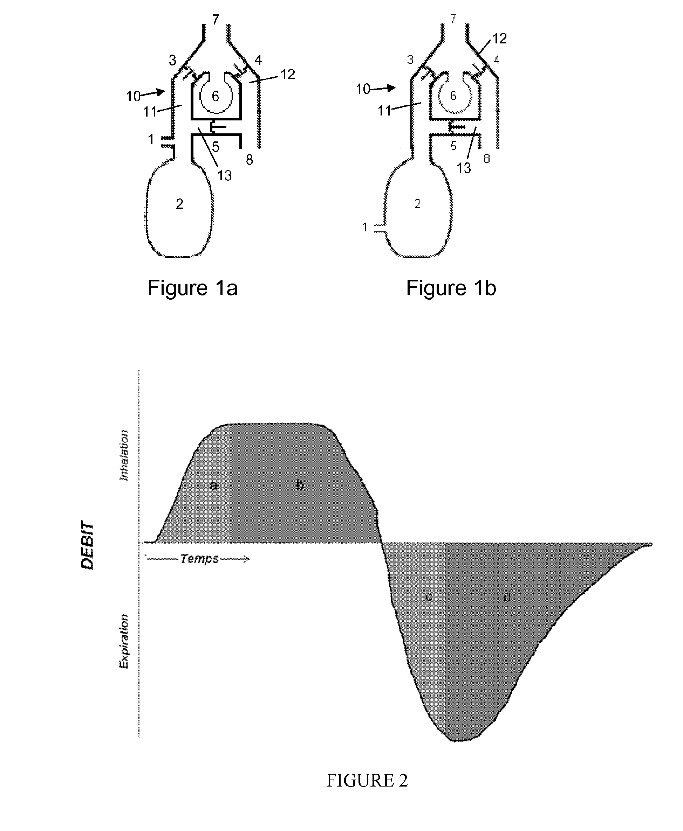

Les Figures 1a et 1b schématisent une architecture de principe de deux modes de réalisation possibles d'un dispositif respiratoire selon l'invention faisant office de distributeur et collecteur de gaz médical.

De façon générale, le dispositif respiratoire de l'invention vient s'insérer entre une source de gaz, par exemple un ventilateur respiratoire, une canalisation, un réservoir ou une bouteille de gaz, et une interface patient, c'est-à-dire typiquement un masque nasal ou facial.

Le dispositif respiratoire de l'invention comporte un circuit de gaz en boucle 10, dans lequel circule le gaz, comprenant une première portion de circuit 11 avec une valve inspiratoire 3, une deuxième portion de circuit 12 avec une valve expiratoire 4 et une troisième portion de circuit 13 avec une valve de dilution 5.

Les trois portions de circuit 11, 12, 13 sont reliées fluidiquement les unes aux autres de manière à former le circuit de gaz 10 en boucle.

La valve inspiratoire 3, la valve expiratoire 4 et la valve de dilution 5 sont des valves à sens unique (*one*-*way* en anglais), ce qui permet d'éviter des retours de flux gazeux.

La valve inspiratoire 3 s'ouvre, lors des phases inspiratoires du patient, pour permettre le passage du gaz frais au travers du premier tronçon 11 de circuit en direction du deuxième tronçon 12 de circuit et son passage ensuite au travers de l'orifice de distribution de gaz 7 qui débouche dans l'interface patient, i.e. un masque respiratoire par exemple. A l'inverse, la valve inspiratoire 3 se referme, lors des phases expiratoires du patient.

Par ailleurs, la valve expiratoire 4 s'ouvre, lors des phases expiratoires du patient, pour permettre le passage des gaz expirés au travers du deuxième tronçon 12 de circuit en direction du deuxième tronçon 12 de circuit et son passage ensuite au travers de l'orifice d'évacuation 8 des gaz. A l'inverse, la valve expiratoire 4 se referme, lors des phases inspiratoires du patient.

La valve de dilution 5 permet d'admettre de l'air ambiant dans le circuit 10 seulement lorsque la demande en gaz du patient fait l'objet d'un pic soudain de consommation, c'est-à-dire excède l'apport total en gaz, à savoir l'alimentation en gaz frais et celle en gaz issu du réservoir de gaz expiratoire 6, comme expliqué ci-après. La valve de dilution 5 se referme, lors des phases expiratoires du patient.

L'orifice d'entrée ou orifice d'alimentation en gaz frais 1 est agencé sur le circuit de gaz 10 entre la valve inspiratoire 3 et la valve de dilution 5, soit directement sur la première portion 11 de circuit (Fig 1a), soit au niveau d'un réservoir de gaz frais 2 en communication fluidique avec ladite première portion 11 de circuit (Fig 1b).

Un orifice 7 de distribution de gaz au patient est par ailleurs agencé sur le circuit de gaz 10, entre la valve inspiratoire 3 et la valve expiratoire 4, et permet de distribuer au patient le gaz médical frais, lors des phases inspiratoires, puis de récupérer les gaz expirés par le patient, lors des phases expiratoires. Il se produit donc une circulation des gaz à double sens au sein de cet orifice 7 en fonction de la phase respiratoire considérée, i.e. phase inspiratoire ou expiratoire.

En outre, un orifice 8 d'évacuation de gaz ou de mise à l'atmosphère est agencé sur le circuit de gaz en boucle 10 située entre la valve expiratoire 4 et la valve de dilution 5, de manière à pouvoir expulser à l'atmosphère les gaz expirés par le patient qui sont riches en CO₂ notamment.

Selon l'invention, quel que soit le mode de réalisation considéré, un réservoir de gaz expiratoire 6, avantageusement déformable, est agencé sur le circuit 10 de gaz en étant relié fluidiquement audit circuit de gaz en boucle 10 entre les valves inspiratoire 3 et expiratoire 4.

L'utilisation d'un tel réservoir de gaz expiratoire 6, préférentiellement déformable, connecté au circuit de gaz en boucle 10 entre lesdites valves inspiratoire 3 et expiratoire 4, préférentiellement à proximité immédiatement de l'orifice de distribution 7 de gaz au patient par lequel passe les gaz inspirés et expirés, permet de capturer de manière sélective les gaz expirés en tout début de phase expiratoire.

En effet, ces premiers gaz expirés en début de phase expiratoire sont généralement peu chargés en CO₂ mais contiennent à l'inverse une concentration élevée en gaz thérapeutique. Le fait de pouvoir les piéger dans un réservoir de gaz expiratoire 6 permet de les piéger jusqu'à la phase inspiratoire suivante sans qu'ils ne soient totalement rejetés à l'atmosphère via l'orifice d'évacuation de gaz 8 agencé sur le circuit de gaz située entre la valve expiratoire et la valve de dilution.

En outre, le dispositif de l'invention comporte non seulement un premier réservoir de gaz frais 2 en communication fluidique avec la première portion 11 de circuit (cf. Fig 1b) et positionné en amont de la vanne inspiratoire 3 mais aussi un deuxième réservoir de gaz expiratoire 6 déformable, encore appelé réservoir-tampon 6, agencé en étant relié fluidiquement au circuit de gaz 10 entre les valves inspiratoire 3 et expiratoire 4, donc près de la connexion dudit circuit 10 avec l'interface patient, c'est-à-dire le masque respiratoire ou analogue, via l'orifice de distribution 7.

Le premier réservoir de gaz frais 2 se remplit de gaz frais, c'est-à-dire de gaz médical ou thérapeutique, pendant les phases expiratoires et peut se vider en fonction des besoins du patient, c'est-à-dire de la demande en gaz du patient, durant les phases inspiratoires.

Le deuxième réservoir de gaz expiratoire 6 déformable ou réservoir-tampon se remplit préférentiellement avec les premiers gaz expirés, lors des débuts de phases expiratoires, et se vide lors des phases inspiratoires. Ces premiers gaz expirés sont encore riches en gaz thérapeutique.

Le seuil de pression minimale permettant l'ouverture de la valve respiratoire 3, lors des phases inspiratoires, est supérieur à la différence de pression entre la pression régnant dans le réservoir-tampon 6 et celle dans l'interface patient requise pour vider le réservoir-tampon 6, de préférence un réservoir à paroi déformable.

Lors des phases inspiratoires, le réservoir-tampon 6 est le premier à se vider, puis ensuite la valve inspiratoire 3 s'ouvre et permet l'alimentation du patient en gaz frais comme illustré en Figure 2, via l'orifice d'entrée et d'alimentation en gaz frais 1 qui est agencé sur le circuit de gaz 10 entre la valve inspiratoire 3 et la valve de dilution 5, c'est-à-dire directement sur la première portion 11 de circuit (Fig 1a) ou alors au niveau du réservoir de gaz frais 2 (Fig 1 b) alimentant la première portion 11 de circuit.

La pression minimale nécessaire à l'ouverture de la valve expiratoire 4 est supérieure à la pression nécessaire pour re-remplir le réservoir-tampon 6 faisant office de réserve de gaz de réinhalation, de sorte que, pendant les phases expiratoires, le réservoir-tampon 6 se remplisse en premier et avant toute ouverture de la valve expiratoire 4, ladite valve expiratoire 4 ne s'ouvrant que lorsque le réservoir-tampon 6 est plein et qu'un éventuel excès de gaz expirés ne bipasse ledit réservoir-tampon 6 et ne soit évacué vers l'atmosphère ambiante, comme schématisé en Figure 2.

Par ailleurs, la pression minimale nécessaire à l'ouverture de la valve de dilution 5 est supérieure à la pression nécessaire pour vider à la fois le réservoir-tampon 6 et le premier réservoir de gaz frais 2, tel un ballon respiratoire. En fait, la valve de dilution 5 agit en tant que sécurité ou valve d'anti-suffocation, laquelle ne s'ouvre et ne laisse passer du gaz que lorsque la demande en gaz du patient ne peut être satisfaite au travers de la totalité du gaz disponible via l'alimentation en gaz frais arrivant par l'orifice d'entrée 1 combinée aux flux de gaz provenant du réservoir-tampon 6 et du premier réservoir de gaz frais 2.

Le patient est quant à lui raccordé au dispositif de l'invention par l'intermédiaire d'une interface appropriée, de préférence un masque respiratoire facial, qui comporte un coussin déformable qui s'adapte aux contours faciaux du patient de sorte qu'un joint étanche soit formé entre le masque et la face du patient, minimisant ainsi les passages inopinés de gaz entre le masque et la face du patient, notamment les fuites de gaz thérapeutique ou, à l'inverse, les entrées d'air ambiant. Ainsi, lorsque la différence de pression entre l'intérieur du masque et l'atmosphère ambiante est égale à la pression minimale de la valve inspiratoire 3, l'entrainement d'air ambiant est inférieur à 5 l/min, avantageusement inférieur à 1 l/min.

Il est à noter que le réservoir-tampon 6 a préférentiellement un volume total égal au volume mort anatomique du patient additionné des éventuels volumes morts de l'interface utilisée, c'est à dire du masque facial.

En fait, de façon générale, le volume total du réservoir-tampon 6 peut être fixé à une valeur moyenne déterminée à partir des volumes morts anatomiques mesurés dans une population donnée de patients, et des volumes morts connus des interfaces existantes, lesquels sont aisément déterminables par de simples mesures de routine.

Par exemple, pour un patient adulte, le volume total du réservoir-tampon 6 est typiquement compris entre 50 et 400 ml. Pour un enfant ou un bébé, volume total du réservoir-tampon 6 peut être inférieur à ces valeurs.

Le volume total du réservoir-tampon 6 peut donc être fixé une fois pour toutes en usine par exemple à partir de données moyennes, comme expliqué ci-dessus, ou alors on peut aussi utiliser un réservoir-tampon 6 à volume variable et ajustable, par exemple par incréments de 50 ml entre 50 et 400 ml, de manière à adapter le volume du réservoir-tampon 6 au cas par cas, c'est-à-dire en fonction de l'anatomie de chaque patient. Il va de soi que les incréments peuvent être supérieurs ou inférieurs à 50 ml et que la plage de réglage peut débuter à une valeur minimale (borne basse) inférieure ou supérieure à 50 ml et se terminer par ailleurs à une valeur maximale (borne haute) inférieure ou supérieure à 400 ml.

Afin d'améliorer encore la sécurité, un dispositif d'adsorption du CO₂ à faible résistance de flux peut être placé immédiatement en amont du réservoir-tampon 6 de sorte d'éliminer tout ou partie du CO₂ expiré par le patient et éviter sa ré-inhalation par la patient lors de la phase inspiratoire suivante. Dans ce cas, la résistance au flux gazeux de ce dispositif doit être aussi faible que possible pour ne pas impacter négativement ou gêner l'ouverture normale des valves inspiratoires 3 et expiratoires 4, comme expliqué ci-avant.

Par ailleurs, le premier réservoir de gaz frais 2 peut comporter une valve de surcharge à sens unique (non schématisée), c'est-à-dire une valve de sécurité qui s'ouvre dans le premier réservoir de gaz frais 2 est totalement rempli de gaz frais jusqu'à atteindre sa capacité maximale en gaz de manière à évacuer tout excès de gaz vers l'atmosphère.

En outre, le dispositif de l'invention peut aussi comprendre une entrée ou connexion additionnelle, positionnée dans l'espace entre le réservoir-tampon 6 et la connexion à l'interface patient, au travers de laquelle un médicament aérosol peut être introduit dans le flux gazeux, par exemple via un aérosol doseur de liquide pressurisé (*pressurized metered-dose inhaler* en anglais), un nébuliseur par jet ou à membrane vibrante.

De façon alternative, un tel aérosol peut aussi être administré via l'alimentation en gaz frais, c'est à dire le port d'entrée 1 des Figures 1a et 1b.

Enfin, le dispositif de l'invention peut aussi comprendre un ou des ports d'échantillonnage ou de test, positionnés sur l'interface patient, tel le masque, ou entre le réservoir-tampon 6 et la sortie 7 reliée à l'interface patient, de sorte de permettre un prélèvement d'environ de 100 à 500 ml/min de gaz qui est extrait du circuit et envoyé vers un appareil de mesure de concentration de gaz, par exemple de mesure de la teneur en l'un ou plusieurs des composés du groupe formé par He, Xe, Ar, N2O, 02, CO2 et/ou vapeur d'eau (humidité), et/ou de mesure de température du gaz

La Figure 2 schématise un cycle respiratoire idéal chez un patient alimenté en gaz par un dispositif à réservoir-tampon 6 selon la présente invention.

Plus précisément, on y voit le débit respiratoire pendant les phases inspiratoires et expiratoires, pour le cas où la demande en gaz du patient égale la quantité de gaz disponible, à savoir :
- la phase a) correspond au début d'inspiration où du gaz provenant du réservoir-tampon 6 est inhalé,
- la phase b) représente la moitié et la fin d'inspiration où du gaz provenant de l'alimentation en gaz frais 1 et/ou du premier réservoir 2 (ballon) est inhalé,
- la phase c) correspond au début d'expiration où du gaz expiré est envoyé vers le réservoir-tampon 6, de préférence du gaz préalablement débarrassé du CO₂,
- la phase d) représente la moitié et la fin d'expiration où gaz expiré riche en CO₂ est envoyé vers l'atmosphère.

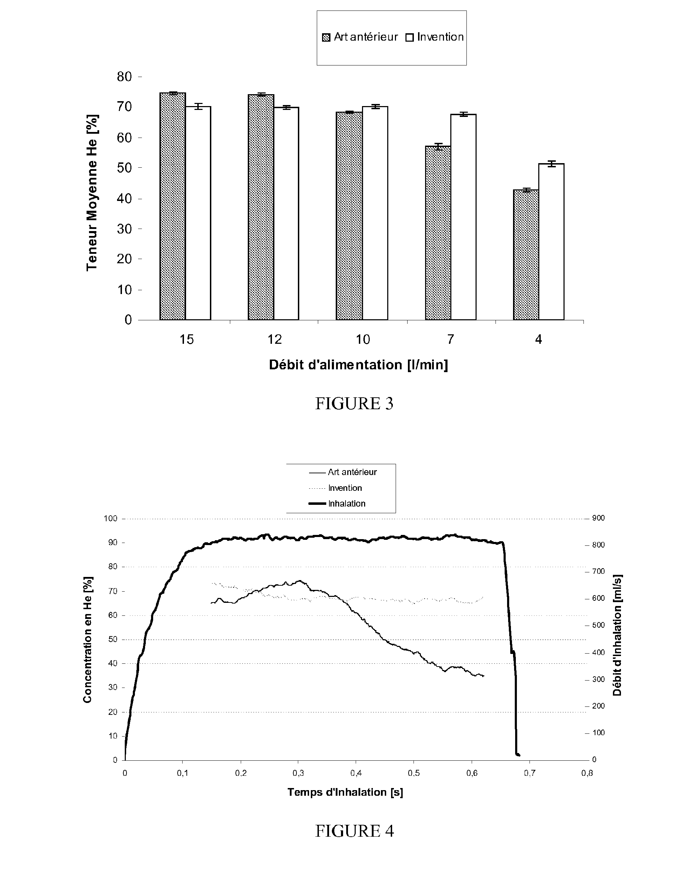

Par ailleurs, la Figure 3 montre les résultats de expériences en laboratoire évaluant la concentration moyenne (en % en volume) d'hélium qui serait administrée à un patient en utilisant un dispositif du commerce sans réservoir-tampon et, à titre comparatif, un dispositif à réservoir-tampon 6 selon la présente invention (cf. Figure 1a) et ce, pour des débits d'alimentation en gaz (mélange hélium/O₂ 78/22% vol.) compris entre 4 et 15 l/min.

Le volume du réservoir-tampon a été maintenu constant à 260 ml. La respiration a été simulée sur une base de 30 respirations par minute avec un volume cyclique (*tidal volume* en anglais) de 500 ml, ce qui correspond à un volume-minute de 15 l/min.

Des flux de gaz d'alimentation entre 4 et 15 l/min correspondent à un rapport alimentation/demande, c'est-à-dire flux d'alimentation / volume minute, de 0,27 à 1.

Les colonnes sur la Figure 3 représentent les valeurs moyennes mesurées sur 7 cycles inspiratoires et les barres d'erreur les surmontant représentent les écarts-types.

Comme on le voit, la concentration en hélium réellement administrée au patient est nettement supérieure avec le dispositif selon l'invention pour les bas débits, i.e. < 12 l/min, correspondant à des rapports alimentation/demande inférieurs à 0.80.

De là, quand la demande du patient excède l'alimentation en gaz frais, le dispositif de l'invention peut être utilisé pour augmenter la concentration en gaz thérapeutique inhalé par le patient.

Par contre, à partir de 12 l/min, la tendance s'inverse car de petites quantité d'air atmosphérique entrent dans le système pendant l'inhalation et pénètrent dans le réservoir-tampon pendant l'expiration, puis sont réinhalées à l'inspiration suivante. Cependant, cet effet peut être évité ou minimisé en améliorant l'étanchéité du système.

Le dispositif selon l'invention est donc particulièrement adapté aux situations dans lesquelles la demande patient excède l'alimentation en gaz frais ou lorsque la demande patient varie au cours du temps, puisqu'il permet de réduire les variations de concentration de gaz médical inhalé par le patient.

La Figure 4 représente la concentration d'hélium (% en volume en ordonnées) mesurée en temps réel (temps en abscisses en secondes), pendant l'inspiration, avec administration d'un débit constant (7 l/min) de mélange gazeux He/O2 (correspondant à un rapport alimentation/demande de 0.47) au moyen d'un dispositif selon la présente invention et, à titre comparatif, d'un dispositif selon l'art antérieur dépourvu du réservoir-tampon 6. Sur la Figure 4, on a aussi représenté le débit inhalatoire instantané.

Les valeurs données sont des moyennes sur 7 cycles inspiratoires.

Avec les dispositifs existants, la concentration en hélium délivrée chute rapidement durant la dernière moitié de la phase inspiratoire du fait de l'ouverture de la vanne de dilution engendrant une entrée d'air de dilution.

A l'inverse, avec le dispositif de l'invention, on peut limiter ces entrées d'air atmosphérique et ainsi éviter des dilutions intempestives du gaz thérapeutique fourni au patient.

## Revendications

1. Dispositif de distribution de gaz comprenant :
- un circuit de gaz en boucle (10) comprenant une première portion de circuit (11) reliée fluidiquement à une deuxième portion de circuit (12), ladite deuxième portion de circuit (12) étant reliée à une troisième portion de circuit (13), et ladite troisième portion de circuit (13) étant reliée fluidiquement à la première portion de circuit (11),
- la première portion de circuit (11) comprenant une valve inspiratoire (3), la deuxième portion de circuit (12) comprenant une valve expiratoire (4), et la troisième portion de circuit (13) comprenant une valve de dilution (5),
- un orifice d'alimentation en gaz frais (1) agencé sur le circuit de gaz (10) entre la valve inspiratoire (3) et la valve de dilution (5),
- un orifice (7) de distribution de gaz au patient agencé sur le circuit de gaz (10) entre la valve inspiratoire (3) et la valve expiratoire (4), et
- un orifice (8) d'évacuation de gaz agencé sur le circuit de gaz (10) entre la valve expiratoire (4) et la valve de dilution (5),
**caractérisé en ce qu'**un réservoir-tampon de gaz expiratoire (6) est relié fluidiquement audit circuit de gaz (10) entre les valves inspiratoire (3) et expiratoire (4).

2. Dispositif selon la revendication 1, **caractérisé en ce qu'**il comprend en outre un réservoir-tampon de gaz frais (2) relié fluidiquement audit circuit de gaz (10) entre les valves inspiratoire (3) et de dilution (5).

3. Dispositif selon l'une des revendications précédentes, **caractérisé en ce que** la valve inspiratoire (3), la valve expiratoire (4) et la valve de dilution (5) sont des valves à sens unique.

4. Dispositif selon l'une des revendications précédentes, **caractérisé en ce que** l'orifice (7) de distribution de gaz est en communication fluidique avec une interface patient, en particulier un masque respiratoire nasal ou facial.

5. Dispositif selon l'une des revendications précédentes, **caractérisé en ce que** le réservoir-tampon de gaz expiratoire (6) est déformable.

6. Dispositif selon l'une des revendications précédentes, **caractérisé en ce que** le réservoir-tampon de gaz expiratoire (6) a un volume interne maximal compris entre 10 et 500 ml, de préférence entre 50 et 400 ml.

7. Dispositif selon l'une des revendications 1 à 5, **caractérisé en ce que** le réservoir-tampon de gaz expiratoire (6) a un volume interne ajustable compris entre 10 et 500 ml, de préférence entre 50 et 400 ml.

8. Dispositif selon la revendication 7, **caractérisé en ce que** le réservoir-tampon de gaz expiratoire (6) a un volume interne ajustable par incréments compris entre 10 et 100 ml, typiquement entre 20 et 60 ml.

9. Installation d'administration de gaz thérapeutique à un patient comprenant une source de gaz thérapeutique alimentant, via l'orifice d'alimentation en gaz frais (1), le circuit de gaz en boucle (10) d'un dispositif de distribution de gaz selon l'une des revendications 1 à 8, et une interface patient connectée fluidiquement à l'orifice (7) de distribution de gaz agencé sur ledit circuit de gaz en boucle (10).

10. Installation selon la revendication 9, **caractérisée en ce que** la source de gaz thérapeutique est un ventilateur et/ou l'interface est un masque respiratoire.

## Patentansprüche

1. Vorrichtung für die Abgabe von Gas, die Folgendes umfasst:
- einen geschlossenen Gaskreislauf (10), der einen ersten Kreislaufabschnitt (11) umfasst, der mit einem zweiten Kreislaufabschnitt (12) fluidisch verbunden ist, wobei der zweite Kreislaufabschnitt (12) mit einem dritten Kreislaufabschnitt (13) verbunden ist und wobei der dritte Kreislaufabschnitt (13) mit dem ersten Kreislaufabschnitt (11) fluidisch verbunden ist,
- wobei der erste Kreislaufabschnitt (11) ein Einatmungsventil (3) umfasst, wobei der zweite Kreislaufabschnitt (12) ein Ausatmungsventil (4) umfasst und wobei der dritte Kreislaufabschnitt (13) ein Verdünnungsventil (5) umfasst,
- eine Öffnung für die Versorgung mit Frischluft (1), die in dem Gaskreislauf (10) zwischen dem Einatmungsventil (3) und dem Verdünnungsventil (5) vorgesehen ist,
- eine Öffnung (7) für die Abgabe von Gas an den Patienten, die in dem Gaskreislauf (10) zwischen dem Einatmungsventil (3) und dem Ausatmungsventil (4) vorgesehen ist, und
- eine Öffnung (8) für den Auslass von Gas, die in dem Gaskreislauf (10) zwischen dem Ausatmungsventil (4) und dem Verdünnungsventil (5) vorgesehen ist,
**dadurch gekennzeichnet, dass** ein Atemgas-Pufferspeicher (6) mit dem Gaskreislauf (10) zwischen dem Einatmungsventil (3) und Ausatmungsventil (4) fluidisch verbunden ist.

2. Vorrichtung nach Anspruch 1, **dadurch gekennzeichnet, dass** sie ferner einen Frischluft-Pufferspeicher (2) umfasst, der mit dem Gaskreislauf (10) zwischen dem Einatmungsventil (3) und Verdünnungsventil (5) fluidisch verbunden ist.

3. Vorrichtung nach einem der vorhergehenden Ansprüche, **dadurch gekennzeichnet, dass** das Einatmungsventil (3), das Ausatmungsventil (4) und das Verdünnungsventil (5) Einwegventile sind.

4. Vorrichtung nach einem der vorhergehenden Ansprüche, **dadurch gekennzeichnet, dass** die Öffnung (7) für die Abgabe von Gas mit einer Patientenschnittstelle, insbesondere einer Nasen- oder Gesichtsatemmaske, in Strömungsverbindung steht.

5. Vorrichtung nach einem der vorhergehenden Ansprüche, **dadurch gekennzeichnet, dass** der Atemgas-Pufferspeicher (6) verformbar ist.

6. Vorrichtung nach einem der vorhergehenden Ansprüche, **dadurch gekennzeichnet, dass** der Atemgas-Pufferspeicher (6) ein maximales Innenvolumen zwischen 10 und 500 ml, vorzugsweise zwischen 50 und 400 ml, hat.

7. Vorrichtung nach einem der Ansprüche 1 bis 5, **dadurch gekennzeichnet, dass** der Atemgas-Pufferspeicher (6) ein einstellbares Innenvolumen zwischen 10 und 500 ml, vorzugsweise zwischen 50 und 400 ml, hat.

8. Vorrichtung nach Anspruch 7, **dadurch gekennzeichnet, dass** der Atemgas-Pufferspeicher (6) ein schrittweise einstellbares Innenvolumen zwischen 10 und 100 ml, typischerweise zwischen 20 und 60 ml, hat.

9. Anlage zur Verabreichung von therapeutischem Gas an einen Patienten, umfassend eine Quelle für therapeutisches Gas, die über die Öffnung für die Versorgung mit Frischluft (1) den geschlossenen Gaskreislauf (10) einer Vorrichtung für die Abgabe von Gas nach einem der Ansprüche 1 bis 8 versorgt, und eine Patientenschnittstelle, die mit der Öffnung (7) für die Abgabe von Gas, die in dem geschlossenen Gaskreislauf (10) vorgesehen ist, fluidisch verbunden ist.

10. Anlage nach Anspruch 9, **dadurch gekennzeichnet, dass** die Quelle für therapeutisches Gas ein Lüfter ist und/oder die Schnittstelle eine Atemmaske ist.

## Claims

1. Gas distribution device comprising:
- a looped gas circuit (10) comprising a first circuit portion (11) which is in fluid communication with a second circuit portion (12), said second circuit portion (12) being connected to a third circuit portion (13), and said third circuit portion (13) being in fluid communication with the first circuit portion (11),
- the first circuit portion (11) comprising an inhalation valve (3), the second circuit portion (12) comprising an exhalation valve (4), and the third circuit portion (13) comprising a dilution valve (5),
- an orifice (1) for supplying fresh gas, which is arranged on the gas circuit (10) between the inhalation valve (3) and the dilution valve (5),
- an orifice (7) for distributing gas to the patient, which is arranged on the gas circuit (10) between the inhalation valve (3) and the exhalation valve (4), and
- an orifice (8) for evacuating gas, which is arranged on the gas circuit (10) between the exhalation valve (4) and the dilution valve (5),
**characterised in that** an exhalation gas buffer tank (6) is in fluid communication with said gas circuit (10) between the inhalation valve (3) and exhalation (4) valve.

2. Device according to claim 1, **characterised in that** it further comprises a fresh gas buffer tank (2) which is in fluid communication with said gas circuit (10) between the inhalation valve (3) and dilution (5) valves.

3. Device according to either of the preceding claims, **characterised in that** the inhalation valve (3), the exhalation valve (4) and the dilution valve (5) are one-way valves.

4. Device according to any of the preceding claims, **characterised in that** the gas distribution orifice (7) is in fluid communication with a patient interface, in particular a nasal or facial respirator mask.

5. Device according to any of the preceding claims, **characterised in that** the exhalation gas buffer tank (6) is deformable.

6. Device according to any of the preceding claims, **characterised in that** the exhalation gas buffer tank (6) has a maximum internal volume of between 10 and 500 ml, preferably of between 50 and 400 ml.

7. Device according to any of claims 1 to 5, **characterised in that** the exhalation gas buffer tank (6) has an adjustable internal volume of between 10 and 500 ml, preferably of between 50 and 400 ml.

8. Device according to claim 7, **characterised in that** the exhalation gas buffer tank (6) has an incrementally adjustable internal volume of between 10 and 100 ml, typically of between 20 and 60 ml.

9. Installation for administering therapeutic gas to a patient, comprising a therapeutic gas source which feeds, via the orifice (1) for supplying fresh gas, the looped gas circuit (10) of a gas distribution device according to any of claims 1 to 8, and a patient interface which is in fluid communication with the gas distribution orifice (7) arranged on said looped gas circuit (10).

10. Installation according to claim 9, **characterised in that** the therapeutic gas source is a ventilator and/or the interface is a respirator mask.
